# EUROPÄISCHE PATENTANMELDUNG

(11) **EP 2 954 912 A1**
(43) Veröffentlichungstag der Anmeldung: **16.12.2015**
(21) Anmeldenummer: 14172416.1
(22) Anmeldetag: 13.06.2014
(51) Int. Cl.: A61M 5/00, A61M 37/00

(54) **Modul für ein Handgerät zum Einbringen einer Substanz durch die Hautoberfläche, Artikel und Anordnung**

(71) Anmelder: MT.DERM GmbH, 14167 Berlin (DE)
(72) Erfinder: Jarling, Christian, Reinhold, 12307 Berlin (DE); Röthig, Sebastian, 10553 Berlin (DE)
(74) Vertreter: Bittner, Thomas L.

(57) **Zusammenfassung**

Die Anmeldung betrifft ein Modul (21) für ein Handgerät (1) zum Einbringen einer Substanz durch die Haut, mit einem Modulgehäuse (22), einer Stecheinrichtung, bei der ein Bauteil (29) in dem Modulgehäuse (22) entlang einer Bauteilführung zwischen einer ersten und einer zweiten Endstellung verlagerbar ist, einem Substanzanschluss (35), einer Fluidverbindung (34), einem Antriebsanschluss (32), und einer der Fluidverbindung (34) zugeordneten Fluidsteuereinrichtung (39), die in dem Modulgehäuse (22) angeordnet und eingerichtet ist, die Fluidverbindung (34) wenigstens dann zu öffnen, wenn das Bauteil (29) in der ersten Endstellung angeordnet ist, und die Fluidverbindung (34) im Vergleich zum Öffnen teilweise oder ganz zu schließen, wenn das Bauteil (29) in der zweiten Endstellung angeordnet ist. (Fig. 1)

## Beschreibung

Die Erfindung betrifft ein Modul für ein Handgerät zum Einbringen einer Substanz durch die Hautoberfläche, einen Artikel sowie eine Anordnung.

### Hintergrund

Es sind Handgeräte bekannt, mit denen eine beliebige Substanz, sei es zum Beispiel zu medizinischen oder kosmetischen Zwecken, durch eine menschliche oder eine tierische Haut im Körper eingebracht werden kann, indem die Haut mittels einer oder mehreren Kanülen, durch deren inneres Strömungsvolumen die Substanz eingedrückt wird, aufgestochen wird. Das Einbringen der Substanz kann in Abhängigkeit von der Stechtiefe in unterschiedliche Hautschichten erfolgen. Die durch die Haut einzubringende Substanz kann hierbei mit einem Druck beaufschlagt werden, um die Substanz aus einem Substanzreservoir in die Kanüle(n) zu drücken, so dass über die Kanülenspitze die Substanz in dem Körper eingebracht wird, insbesondere in tieferliegender Schichten der Haut. Ein solches Einbringen der Substanz kann auch als Injizieren bezeichnet werden.

Aus dem Dokument EP 2 633 873 A1 ist zur Injektion eines Arzneistoffes in Verbindung mit einer Hauttherapie bekannt. Bei der bekannten Vorrichtung wird auf den Ausgang eines als Spritze ausgeführten Substanzreservoirs eine Modulspitze mit einer Kanüle aufgesetzt, durch welche hindurch dann die Substanz in der Haut eingebracht wird. Das Substanzreservoir ist mit Hilfe eines Schwenkbügels an der Vorrichtung befestigt.

Eine Vorrichtung mit teilweisen ähnlichen technischen Merkmalen ist in dem Dokument US 8,118,779 B2 offenbart.

Eine weitere Vorrichtung zur Injektion eines Fluides oder eines Gels ist im Dokument US 2011/0230833 A1 offenbart.

Im Dokument EP 2 633 882 A1 ist ein Modul beschrieben, welches in Verbindung mit Behandlungen einer Haut zum Einsatz kommen soll. Bei dem bekannten Modul ist in einem Modulgehäuse eine Stecheinrichtung mit einer Kanülenanordnung aufgenommen, die an einem hin und her bewegbaren Bauteil angeordnet ist. Im Betrieb wird das Bauteil mit Hilfe einer pneumatisch erzeugten Antriebskraft vor- und zurückbewegt, derart, dass im ausgefahrenen Zustand Kanülenspitzen zugeordneten Gehäuseaustrittsöffnungen vorgelagert sind. Die pneumatische Antriebskraft wird über einen Gehäuseanschluss in das Modulgehäuse eingekoppelt. Das Modul ist zum Anschluss an eine Spritze vorgesehen, mit der ein Substanzreservoir bereitgestellt ist. Das Lumen der Kanülen steht mit einem Hohlraum in Verbindung, welcher seinerseits an das Substanzreservoir koppelt, wenn das Modul auf die Spritze aufgesteckt ist. Das Modulgehäuse ist mehrteilig ausgefiihrt. Ein vorderer Gehäuseabschnitt, in welchem die Austrittsöffnungen für die Kanülen gebildet sind, ist über eine Schraubverbindung mit einem rückwärtigen Gehäuseteil verbunden, welches seinerseits zum Aufstecken auf die Spritze dient.

### Zusammenfassung

Aufgabe der Erfindung ist es, verbesserte Technologien im Zusammenhang für ein Handgerät zum Einbringen einer Substanz durch die Hautoberfläche einer menschlichen oder tierischen Haut anzugeben, bei denen die zielgerichtete Dosierung der einzubringenden Substanz erleichtert ist.

Zur Lösung der Aufgabe ist ein Modul für ein Handgerät zum Einbringen einer Substanz durch die Hautoberfläche nach dem unabhängigen Anspruch 1 geschaffen. Weiterhin sind ein Artikel nach dem unabhängigen Anspruch 12 sowie eine Anordnung nach dem unabhängigen Anspruch 13 vorgesehen. Vorteilhafte Ausgestaltungen sind Gegenstand von abhängigen Unteransprüchen.

Nach einem Aspekt ist ein Modul für ein Handgerät zum Einbringen einer Substanz durch die Hautoberfläche geschaffen, wahlweise durch obere Schichten der Haut oder die Haut insgesamt. Das Handgerät, für welches das Modul bestimmt ist, kann auch als Handstück bezeichnet werden, insbesondere dann, wenn es an ein externes Steuergerät oder einer externe Steuereinheit koppelt, von dem für den Betrieb bestimmte Steuersignale bereitgestellt werden, deren Spezifikation zuvor vom Nutzer am Steuergerät ausgewählt sein kann.

Das Modul weist ein Modulgehäuse auf, welches eingerichtet ist, lösbar an das Handgerät zu koppeln. Im Modulgehäuse ist eine Stecheinrichtung angeordnet, mit der die menschliche oder tierische Haut zum Einbringen der Substanz aufgestochen oder durchstochen werden kann. Bei der Stecheinrichtung trägt ein Bauteil oder -element eine Kanülenanordnung mit einer oder mehreren Kanülen (Hohlnadeln). Das Bauteil ist im Betrieb in dem Modulgehäuse entlang einer Bauteilführung zwischen einer ersten und einer zweiten Endstellung verlagerbar, derart, dass eine jeweilige Kanülenspitze der einen oder der mehreren Kanülen in zumindest der ersten Endstellung einer Austrittsöffnung des Modulgehäuses außenseitig vorgelagert ist. Es kann in einer Ausführung vorgesehen sein, dass die eine oder die mehreren Kanülenspitzen in der zweiten Endstellung vollständig in das Modulgehäuse eingefahren sind.

Am Modulgehäuse ist weiterhin ein Substanzanschluss gebildet, der eingerichtet ist, beim Anordnen des Moduls an dem Handgerät an ein druckbeaufschlagtes Substanzreservoir mit der durch die Haut einzubringenden Substanz zu koppeln. Über eine Fluidverbindung steht die Kanülenanordnung mit dem Substanzanschluss in Verbindung, so dass die Substanz im Betrieb aus dem Substanzreservoir über den Substanzanschluss in die Fluidverbindung und dann in die eine oder die mehreren Kanülen einspeisbar ist. Hierdurch gelangt die Substanz aus dem Substanzreservoir schließlich in den inneren Hohlraum der Kanüle, der auch als Lumen bezeichnet werden kann. Am Modulgehäuse ist weiterhin ein Antriebsanschluss gebildet, über den eine das Bauteil im Betrieb treibende Antriebskraft einkoppelbar ist.

Der Fluidverbindung zwischen Substanzanschluss und Kanüle(n) ist eine Fluidsteuereinrichtung zugeordnet, die in dem Modulgehäuse angeordnet und eingerichtet ist, die Fluidverbindung wenigstens dann zu öffnen, wenn das Bauteil in der ersten Endstellung angeordnet ist, und die Fluidverbindung im Vergleich zu diesem Öffnen teilweise oder ganz zu schließen, wenn das Bauteil in der zweiten Endstellung angeordnet ist. Das Öffnen der Fluidverbindung kann zum Beispiel derart erfolgen, dass Durchflussvolumen / Durchflussquerschnitt der Fluidverbindung vollständig oder teilweise freigegeben ist, wenn das die Kanülenanordnung tragende Bauteil in die erste Endstellung verlagert ist.

Weiterhin ist ein Artikel vorgesehen, bei dem das Modul in einer Sterilverpackung aufgenommen ist.

Nach einem weiteren Aspekt ist eine Anordnung mit einem Handgerät zum Einbringen einer Substanz durch die Haut und dem Modul vorgesehen, wobei das Modul lösbar an einem Modulanschluss oder in eine Modulaufnahme des Handgerätes angeordnet ist, derart, dass der Substanzanschluss des Moduls an ein druckbeaufschlagtes Substanzreservoir mit der durch die Haut einzubringenden Substanz am Handgerät und der Antriebsanschluss des Moduls an einen Antriebsanschluss funktionell koppeln. Über den Antriebsanschluss wird eine Antriebskraft bereitgestellt, die im Handgerät selbst oder außerhalb des Handgerätes von einer Antriebseinrichtung erzeugt wird.

Mit Hilfe der der Fluidverbindung zugeordneten Fluidsteuereinrichtung ist die Dosierung beim Ausbringen der Substanz über die Kanülenanordnung auf einfache und effiziente Weise steuerbar. Einerseits kann sichergestellt werden, dass nur eine gewünschte Menge der Substanz pro Stechbewegung ausgebracht wird. Darüber hinaus ist unnötiger Substanzverlust vermieden, wenn das die Kanülenanordnung tragende Bauteil in der zweiten Endstellung angeordnet ist, welche einer teilweise oder vollständig eingefahrenen Stellung der Kanülenanordnung entspricht.

Die Fluidverbindung kann in einem Teilabschnitt oder auf ihre gesamte Länge zwischen dem Substanzanschluss und der einen oder den mehreren Kanülen aus einem flexiblen Material gebildet sein. Die Fluidverbindung kann teilweise oder vollständig als Schlauchverbindung ausgeführt sein. Das Ausbilden der Fluidverbindung wenigstens abschnittsweise aus dem flexiblen Material ist eine mögliche Ausführung, um eine Relativbewegung zwischen dem die Kanülenanordnung tragenden Bauteil und dem Substanzanschluss im Betrieb zuzulassen. Das flexible Material kann elastisch sein, so dass eine Streckung und Stauchung eines flexiblen und elastischen Abschnittes der Fluidverbindung ermöglicht ist. Bei dieser oder anderen Ausführungsformen kann die Bewegungsbahn des die Kanülenanordnung tragenden Bauteils im Betrieb in Längsrichtung des Substanzanschlusses und diesem gegenüberliegend oder parallel versetzt hierzu ausgebildet sein. Die Bewegungsbahn des Bauteils kann auch nicht parallel zur Längsrichtung des Substanzanschlusses verlaufen.

Bei einer Ausgestaltung kann vorgesehen sein, dass der Substanzanschluss und / oder der Antriebsanschluss an einer Rückwand des Modulgehäuses angeordnet sind. Der Substanzanschluss und der Antriebsanschluss können im Bereich der gleichen oder an unterschiedlichen Wänden des Modulgehäuses gebildet sein, zum Beispiel kann ein Anschluss an der Rückwand und der andere Anschluss an einer Seitenwand des Modulgehäuses angeordnet sein.

Die Fluidsteuereinrichtung kann mehrteilig ausgeführt sein. Auch eine einteilige Ausführung der Fluidsteuereinrichtung kann vorgesehen sein. Im Fall mehrerer Elemente oder Komponenten der Fluidsteuereinrichtung können diese relativ zueinander verlagerbar sein, sei es quer oder schräg zur Strömungsrichtung im zugeordneten oder benachbarten Abschnitt der Fluidverbindung. Auch eine gegenläufige Verlagerung von Elementen der Fluidsteuereinrichtung kann vorgesehen sein, beispielsweise derart, dass die gegenläufig bewegbaren Elemente oder Bauteile aufeinander zu und voneinander weg bewegt werden, um die Strömung in der Fluidverbindung betriebsbedingt einzustellen und zu steuern. Die Fluidsteuereinrichtung kann teilweise oder vollständig innerhalb der Strömung der Substanz angeordnet sein. Alternativ oder ergänzend können die Fluidsteuereinrichtung oder Elemente hiervon außerhalb der strömenden Substanz angeordnet sein, beispielsweise auf einer oder gegenüberliegend einer äußeren Oberfläche eines Schlauches, welcher Teil der Fluidverbindung ist.

Eine Fortbildung kann vorsehen, dass die Fluidsteuereinrichtung einen Klemmmechanismus aufweist, der eingerichtet ist, die Fluidverbindung zum teilweisen oder vollständigen Schließen abzuklemmen. Bei dem Klemmmechanismus kann es sich beispielsweise um eine Schlauchklemmung handeln. Alternativ oder ergänzend kann die Fluidsteuerung eine Ventileinrichtung zum Öffnen und Schließen der Fluidverbindung aufweisen.

Die Fluidsteuereinrichtung kann mit dem die Kanülenanordnung tragenden Bauteil in Wirkverbindung stehen. Mit Hilfe der Wirkverbindung wird einer Ausführungsform ein Betätigen der Fluidsteuereinrichtung erzwungen aufgrund der Verlagerung des die Kanülenanordnung tragenden Bauteils zwischen den beiden Endstellungen. Die Wirkverbindung kann mit Hilfe einer mechanischen, einer magnetischen und / oder einer elektromagnetischen Kopplung zwischen Bauteil und Fluidsteuereinrichtung ausgebildet sein. Komponenten oder Elemente der Fluidsteuereinrichtung können an dem Bauteil angeordnet sein, beispielsweise hierauf sitzend oder lagernd. Die Wirkverbindung kann eine zeitgleiche oder zeitverzögerte Betätigung der Fluidsteuereinrichtung in Beziehung zur Verlagerung des die Kanülenanordnung tragenden Bauteils bewirken. Die Wirkverbindung zwischen dem Bauteil und der Fluidsteuerung kann fest oder lösbar ausgeführt sein.

Die Fluidverbindung kann gegenüber einem rückseitigen Kanülenanschluss in Längsrichtung der zugeordneten Kanüle verlaufen. Über den rückseitigen Kanülenanschluss koppelt die Fluidverbindung an den inneren Hohlraum der zugeordneten Kanüle(n), um so die Substanz in die Kanüle(n) einzuleiten. Im Sinne einer rückseitigen Verlängerung des inneren Hohlraums der Kanüle verläuft die Fluidverbindung, beispielsweise in einem geführten Schlauch oder in einem Kanal in dem die Kanülenanordnung tragenden Bauteil, in Längsrichtung der zugeordneten Kanüle. Auf diese Weise können zum Beispiel Biegungen oder Ecken im Bereich benachbart zum rückseitigen Kanülenanordnung vermieden werden.

Eine Weiterbildung kann vorsehen, dass an dem die Kanülenanordnung tragenden Bauteil ein Kolben angeordnet ist, welcher sich beim Verlagern zwischen der ersten und der zweiten Endstellung zumindest abschnittsweise in einer Kolbenführung bewegt. Kolben und Kolbenführung können Bestandteil eines pneumatischen Kolben-Führungs-Systems sein, bei dem der Kolben in der Kolbenführung mittels pneumatischen Drucks bewegt wird. Hierbei wird der pneumatische Druck (Über- und / oder Unterdruck) über den Antriebsanschluss eingekoppelt, so dass eine Antriebskraft bereitgestellt ist. Bei einer anderen Ausführungsform kann es sich um ein magnetisches oder elektrisches Kolben-Führungs-System handeln, bei dem zum Beispiel elektrische Signale über den Antriebsanschluss eingekoppelt werden. Beispielsweise kann die Kolbenbewegung mittels einer Spule erzeugt werden, in welcher ein Magnet mittels Anlegen von Strom an die Spule bewegt wird, derart, dass die spuleninduzierte Bewegung des Magneten auf den Kolben übertragen wird.

Der Antriebsanschluss kann einen pneumatischen Anschluss aufweisen.

Das Modulgehäuse kann mehrteilig mit einem Modulgehäusekörper und einer Modulgehäusespitze ausgeführt sein, die am Modulgehäusekörper und relativ hierzu verlagerbar angeordnet ist. Die Gehäuseaustrittsöffnungen, durch welche sich die Kanülen im Betrieb vor und zurück bewegen können an der Modulgehäusespitze gebildet sein. Mit Hilfe der Relatiwerlagerung von Modulgehäusespitze und Modulgehäusekörper zueinander kann eine Einstelleinrichtung bereitgestellt sein, mit der der Überstand der Kanülenspitzen in der ersten Endstellung in Beziehung zu den Gehäuseaustrittsöffnungen einstellbar ist. Hierdurch kann eine Einstechtiefe der Kanülenspitzen in die Haut eingestellt werden. Die Rückwand des Modulgehäuses kann am Modulgehäusekörper gebildet sein. Die Modulgehäusespitze kann mit Hilfe einer Gewindeverbindung in den Modulgehäusekörper eingeschraubt sein und kann auf diese Weise in Längsrichtung des Moduls relativ zum Modulgehäusekörper verlagert werden. Auch eine lineare Führung mit einer stufenlosen oder rastenden Klemmung kann in einer anderen Ausgestaltung vorgesehen sein.

Das Modul kann als Einwegmodul ausgeführt sein.

In Verbindung mit der Anordnung, welche das Handgerät oder das Handstück zum Einbringen der Substanz durch die Haut sowie das hieran angeordnete Modul aufweist, kann vorgesehen sein, dass Substanzreservoir lösbar in einer zugeordneten Aufnahme am Handgerätgehäuse angeordnet ist. Beispielsweise kann vorgesehen sein, dass die lösbare Befestigung des Substanzreservoirs an dem Handgerätgehäuse mit Hilfe einer mechanischen Befestigung ausgeführt ist, zum Beispiel einem Schwenkhebel oder -bügel, welcher zwischen einer geöffneten Stellung, in welcher das Substanzreservoir in die zugeordnete Aufnahme eingesetzt und hieraus entnommen werden kann, und einer geschlossenen Stellung schwenkbar ist, in der das Substanzreservoir an dem Handgerätegehäuse fixiert ist. Der Schwenkhebel oder -bügel kann zumindest in der geschlossenen Stellung gesichert sein, zum Beispiel mittels Einrasten. Die Kontaktstelle zwischen Modul und Schwenkhebel kann hierfür modulseitig einen Hinterschnitt aufweisen. Am Schwenkhebel kann eine zugeordnete Nase gebildet sein.

Wenn das Substanz- oder Wirkstoffreservoir in der zugeordneten Aufnahme am Handgerät angeordnet ist, wird im Betrieb die Substanz / der Wirkstoff mit Druck beaufschlagt, indem von außen gegen einen Reservoirkolben gedrückt wird, was dem Wirkprinzip einer Spritze vergleichbar ist. Hierfür kann ein Mechanismus mit einer Schubstange und einer hieran koppelnden Gewindestange vorgesehen sein. Zum Antrieb der Gewindestange kann zwischen einem Motor, der im Handgerätegehäuse aufgenommen ist und eine Antriebsdrehbewegung bereitstellt, und der Gewindestange ein Umlenkgetriebe zur Übertragung der drehenden Antriebsbewegung auf die Gewindestange vorgesehen sein.

Weiterhin kann das Handgerätegehäuse in einer Ausführung einen Pneumatikantrieb aufweisen. Alternativ ist der Pneumatikantrieb außerhalb des Handstücks gebildet. Er dient in den verschiedenen Ausführungen dem Bereitstellen einer pneumatischen Antriebskraft, die im Betrieb auf das lösbar befestigte Modul eingekoppelt wird, um dort das die Kanülenanordnung tragende Bauteil zwischen den Endstellungen hin und her zu bewegen. In der Verbindung zwischen dem Pneumatikantrieb und dem Antriebsanschluss können ein oder mehrere Pneumatikventile angeordnet sein, sei es im und / oder außerhalb des Handgerätes.

Der Motor und / oder der pneumatische Antrieb können an eine Steuereinrichtung koppeln, derart, dass im Betrieb von der Steuereinrichtung jeweilige Steuersignale empfangen werden, insbesondere zum aufeinander abgestimmten und koordinierten Bereitstellen von Motorantriebskraft und pneumatischer Antriebskraft. Die Steuereinrichtung kann teilweise oder insgesamt im Handgerät angeordnet sein, zum Beispiel mit einer integrierten Schaltung auf einer Platine. Alternativ kann die Steuereinrichtung wenigstens teilweise im externen Steuergerät gebildet sein, welches funktionell an das Handgerät koppelt.

Bei der Anordnung mit Handgerät und Modul kann die Steuereinrichtung an eine Druckeinrichtung, welche das Substanzreservoir mit einem Betriebsdruck beaufschlagt, und die Antriebseinrichtung koppeln und eingerichtet sein, jeweilige Steuersignale an die Druckeinrichtung und die Antriebseinrichtung zeitlich koordiniert abzugeben. Alternativ oder ergänzend zum koordinierten Ansteuern der Antriebseinrichtung kann bei einem pneumatischen Antrieb vorgesehen sein, Pneumatikventile in der Verbindung zwischen Antrieb und Antriebsanschluss am Modul anzusteuern.

Bei einer Ausgestaltung kann vorgesehen sein, dass zumindest Teilkomponenten der Steuereinrichtung in einem Handgerätegehäuse angeordnet sind. Die Steuereinrichtung kann auch insgesamt, also mit allen ihren Einzelteilen oder -komponenten, in dem Handgerätegehäuse angeordnet sein.

An das Handgerätegehäuse können ein elektrischer und / oder ein pneumatischer Anschluss herangeführt sein.

In einer Ausgestaltung kann ein Modulverriegelungsbügel vorgesehen sein, welcher zur Positionierung und Arretierung des Moduls am Handgerät dient. Vor dem eigentlichen Zuführen des Moduls ist der Modulverriegelungsbügel in einer geöffneten Stellung. Hierzu wird der Modulverriegelungsbügel um seinen Drehpunkt nach oben geschwenkt, bis er in der Endposition einrastet. Nach dem Einschieben des Moduls in die Modulaufnahme wird der Modulverriegelungsbügel in Gegenrichtung aus der Endposition zurückgeschwenkt, bis er die Verriegelungsposition erreicht hat. Hierbei kann der Modulverriegelungsbügel, wenn nötig, mit seiner Kontaktstelle zum Modul, das Modul weiter in die Modulaufnahme hinein schieben und die Modulrückwand mit den Dichtungselementen in der Modulaufnahme verpressen. Ein Rastelement am Modulverriegelungsbügel kann dafür sorgen, dass der Bügel in Verbindung mit dem Modul in seiner Position fixiert bleibt. Der Modulverriegelungsbügel des Handgerätes kann nach Außen geklappt werden, und das Modul wird durch die entstehende Öffnung in die Modulaufnahme eingesetzt. Anschließend kann der Bügel wieder zurück geklappt werden, bis eine Rastnase des Bügels an die Aufnahme des Moduls einrastet.

Sollte der Bügel oder eine andere Verriegelungseinrichtung für das Modul seine Endposition nicht erreichen, kann die Nutzung des Handgerätes ausgeschlossen sein, da das Druckluftsystem (pneumatischer Antrieb) nicht geschlossen ist und so für die Bewegung des Bauteils mit der Kanüle keine Antriebskraft bereitgestellt ist.

Das Handgerät kann genutzt werden, um beliebige Substanzen oder Wirkstoffe durch die Hautoberfläche im Körper einzubringen, insbesondere auch in Hautschichten selbst. So kann zum Beispiel ein Einsatz bei der sogenannten Mesotherapie vorgesehen sein. Hierbei werden mittels der Kanülen (Hohlnadeln), die Teil einer Mesotherapiepistole sein können, individuell zusammengestellte Medikamente und Wirkstoffe direkt in die Haut im zu behandelnden Bereich injiziert. Die minimalinvasive Methode verbindet Grundlagen der Akupunktur, der Neuraltherapie, der Arzneitherapie und nutzt das Prinzip der Reflexzonen. Die Wirkung der Mesotherapie basiert auf der gezielten lokalen Anwendung von verschiedenen Wirkstoffen sowie dem stimulierenden physikalischen Effekt der Nadelung. Die Mikroinjektion kann in bestimmte Akupunktur- und Reaktionspunkte erfolgen. Hierbei entsteht zum Beispiel ein Hautdepot mit den Wirkstoffen, die nach und nach abgegeben werden, was einen schnellen und gleichzeitig anhaltenden Effekt gewährleistet. Injiziert werden kann beispielweise eine individuell zusammengestellte Kombination verschiedener Arzneien, Vitamine, homöopathischer und / oder pflanzlicher Mittel, die sich gegenseitig verstärken und ergänzen können. Auch eine Injektion der folgenden Stoffe kann vorgesehen sein: dermale Füller, Zellen, DANN, RNA, PRP, Peptide, Hyaluronsäure, Toxine, Impfstoff, und / oder Substanzen zur Liopolyse.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgerätes zum Einbringen einer Substanz durch die Haut im Querschnitt,
- Fig. 2: eine schematische Darstellung eines Moduls für das Handgerät im Querschnitt und
- Fig. 3: eine schematische Darstellung zum Betrieb des Handgerätes.

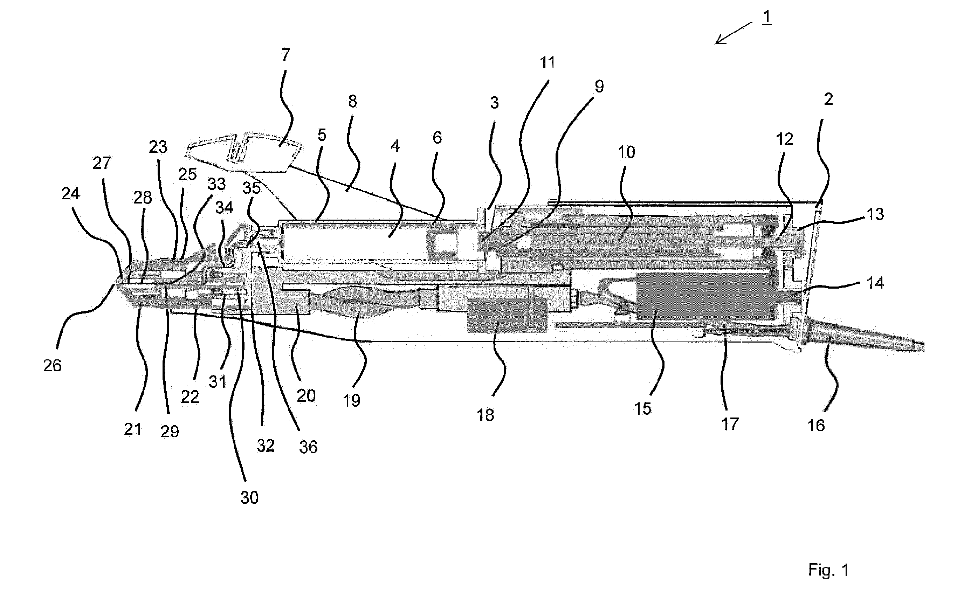

Fig. 1 zeigt eine schematische Darstellung eines Handgerätes 1 zum Einbringen (Injektion) einer Substanz oder eines Wirkstoffes (Verbrauchsmaterial), zum Beispiel als Flüssigkeit oder Gel, durch oder in eine menschliche oder eine tierische Haut. Das Injizieren der Substanz kann beispielsweise einer medizinischen oder einer kosmetischen Behandlung dienen, insbesondere einer Hautbehandlung.

Das Handgerät 1, welches auch als Handstück bezeichnet werden kann, weist ein Gehäuse 2 auf. Das Handgerät oder Handstück 1 kann als eine Baueinheit zur Verfügung gestellt werden, die dem Nutzer zur Verfügung gestellt wird, ohne dass dieser hier Einzelteile zusammenbauen muss.

Am Gehäuse 2 ist eine Aufnahme 3 gebildet, in welcher ein Substanzreservoir 4 lösbar angeordnet ist. Das Substanzreservoir 4 dient dem Bereitstellen der durch die Haut einzubringenden Substanz. Bei der dargestellten Ausführungsform weist das Substanzreservoir 4 ein Reservoirgehäuse 5 sowie einen hierin in Längsrichtung verschiebbaren Reservoirkolben 6 auf. Das Substanzreservoir 4 ist in der Aufnahme 3 lösbar angeordnet und kann hierin mit Hilfe einer Befestigungseinrichtung 7 gesichert, die bei der dargestellten Ausführungsform mit einem Schwenkhebel 8 gebildet ist. Alternativ oder ergänzend kann der Schwenkhebel 8 zur Befestigung eines Moduls dienen, was unten näher erläutert ist.

Um den Betrieb einen Vorschub für den Reservoirkolben 6 in Längsrichtung des Reservoirgehäuses 5 zu erzeugen, ist eine Schubstange 9 vorgesehen, die an eine Gewindestange 10 koppelt, derart, dass mittels Drehen der Gewindestange 10 die Schubstange 9 vor und zurück bewegt werden kann. Wird die Schubstange 9 mittels Drehen der Gewindestange 10 in Richtung des Reservoirkolbens 6 bewegt, wird, nachdem ein distales Ende 11 der Schubstange 9 in Kontakt mit dem Reservoirkolben 6 tritt, Druck auf die Substanz in dem Substanzreservoir 4 ausgeübt, wodurch die Substanz druckbeaufschlagt wird.

Ein proximales Ende 12 der Gewindestange 10 koppelt an ein Umkehr- oder Umlenkgetriebe 13, welches seinerseits an einen Ausgang 14 eines Motors 15 koppelt. Über den Ausgang 14 wird eine drehende Antriebsbewegung bereitgestellt, die über das Umlenkgetriebe 13 auf die Gewindestange 10 übertragen wird, um so schließlich den Reservoirkolben 6 zu bewegen. Eine elektrische Energieversorgung für den Motor 15 ist über eine Anschlussleitung 16 bereitgestellt.

Bei der gezeigten Ausführungsform ist über die Anschlussleitung 16 weiterhin eine pneumatische Versorgungsleitung 17 in das Gehäuse 2 geführt. Die pneumatische Anschlussleitung 17 koppelt an eine Anordnung 18 mit einem oder mehreren Pneumatikventilen, an die sich eine pneumatische Zwischenleitung 19 anschließt. Die pneumatische Zwischenleitung 19 führt zu einer Modulaufnahme 20, in welcher ein Modul 21 lösbar aufgenommen ist. Das Modul 21 kann in einer Ausgestaltung mittels des Schwenkhebels 8 in der Aufnahme 3 gesichert sein, wobei der Schwenkhebel 8 in der geschlossenen Stellung (nicht dargestellt) einrasten kann. Die Kontaktstelle zwischen Modul und Schwenkhebel 8 kann hierfür modulseitig einen Hinterschnitt aufweisen. Am Schwenkhebel 8 kann eine zugeordnete Nase gebildet sein.

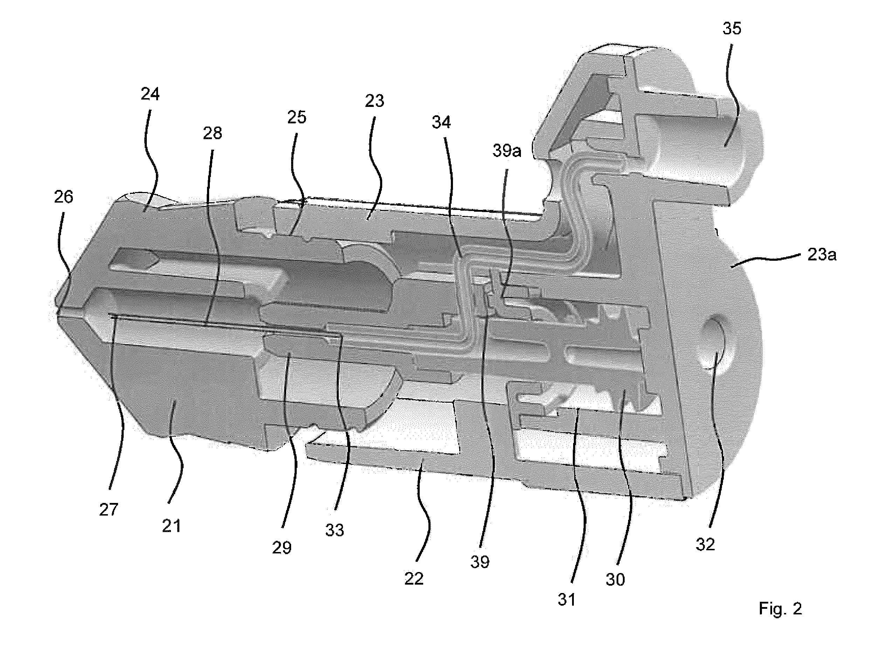

Gemäß Fig. 2 ist das Modul 21 mit einem Modulgehäuse 22 gebildet, welches einen Modulgehäusekörper 23 und eine Modulgehäusespitze 24 sowie einen rückseitigen Modulabschluss 23a aufweist. Die Modulgehäusespitze 24 ist mit Hilfe einer Gewindeverbindung 25 in den Modulgehäusekörper 23 eingeschraubt und kann auf diese Weise in Längsrichtung des Moduls 21 relativ zum Modulgehäusekörper 23 verlagert werden. Hierdurch ist die Relativposition eines vorderseitigen Gehäuseaustritts 26 zur Kanülenspitze 27 einer Kanüle 28 einstellbar. Die Kanüle 28 ist ihrerseits an einem Bauteil 29 aufgenommen, welches rückseitig einen Kolben 30 aufweist, der in einer Kolbenführung 31 in Längsrichtung des Moduls 21 verlagerbar angeordnet ist. Auf den Kolben 30 koppelt über einen am Modul 21 gebildeten Antriebsanschluss 32 die über die pneumatische Zwischenleitung 19 eingekoppelte pneumatische Antriebskraft (vgl. Fig. 1). Hierdurch wird der Kolben 30 in der Kolbenführung 31 zwischen zwei Endstellung vor und zurück bewegt, derart, dass hierdurch die Kanülenspitze 27 ausund eingefahren wird.

Ein rückseitiger Kanülenanschluss 33 steht über eine hier als flexible Schlauchverbindung ausgebildete Fluidverbindung 34 mit einem am Modul 21 gebildeten Substanzanschluss 35 in Verbindung, welcher seinerseits mit einem Reservoirausgang 36 des Substanzreservoirs 4 fluidisch verbunden ist, so dass die Substanz aus dem Substanzreservoir 4 über den Subtanzanschluss 35 und die Fluidverbindung 34 zur Kanüle 28 gelangen kann.

An dem Bauelement ist eine Fluidsteuereinrichtung 39 gebildet, mit der die Fluidverbindung 34 geöffnet und geschlossen wird, indem die Fluidsteuereinrichtung 39 in Abhängigkeit von der Stellung des Kolbens 30 in der Kolbenführung 31 geöffnet und geschlossen wird, wobei ein Ventilbauteil 39a der Fluidsteuereinrichtung 39 hierbei mehr oder weniger gegen die Außenwand der hier mittels eines Schlauch ausgeführten Fluidverbindung 34 drückt, zum Beispiel mittels mehrerer Klemmstege, wodurch der Fluss der Substanz durch die Fluidverbindung 34 einerseits freigegeben und andererseits mehr oder weniger unterbunden wird. Hierdurch ist eine Ausführung eines Ventils zum Öffnen und Schließen der Fluidverbindung 34 gebildet. Andere Ausgestaltungen könne vorgesehen sein, zum Beispiel ein schwenkbar gelagertes Ventilbauteil, welches je nach Schwenkstellung mehr oder weniger von außen auf den Schlauch der Fluidverbindung 34 drückt.

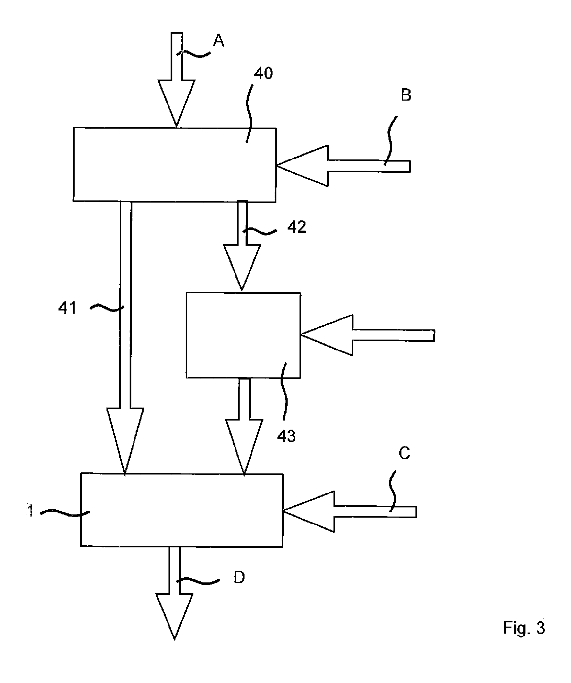

Fig. 3 zeigt eine schematische Darstellung zur Erläuterung einer Betriebsweise des Handgerätes 1. Dieses koppelt gemäß der Darstellung in Fig. 3 an ein Steuergerät 40, welches über eine elektrische Signalleitung 41 sowie eine pneumatische Leitung 42 mit dem Handgerät 1 verbunden ist. In die pneumatische Leitung 42 ist bei der gezeigten Ausführungsform ein Schalter 43 integriert, der zum Beispiel als Fußschalter ausgeführt sein kann. Mit Hilfe des Schalters 43 wird die Zufuhr pneumatischer Energie an das Handgerät 1 freigegeben oder unterbrochen.

Gemäß der schematischen Darstellung in Fig. 3 werden am Steuergerät 40 Betriebsparameter eingestellt, beispielsweise eine Dosiermenge betreffend das Ausbringen der Substanz, eine Frequenz für die Stechbewegung der Kanüle 28 sowie der Beginn und das Ende einer Anwendung. Derartige Steuerparameter können im Steuergerät 40 für unterschiedliche Anwendungszwecke gespeichert werden. Die Eingabe der Bedienparameter ist in Fig. 3 schematisch mit Hilfe eines Pfeils A gezeigt. Ein Pfeil B deutet die Bereitstellung von elektrischer Energie und pneumatischer Energie über das Steuergerät 40 an. Pfeil C zeigt in Fig. 3 schematisch die Bereitstellung / Zufuhr der auszubringenden Substanz. Pfeil D symbolisiert die Bewegung der Kanüle 28, wahlweise auch repetierend, zum dosierten Ausbringen der Substanz.

Nachfolgend werden weitere Aspekte in Verbindung mit dem Handgerät 1 und dessen Betrieb näher erläutert.

Die Versorgung mit Druckluft (pneumatischer Antrieb) kann mittels Kompressoren oder durch bereits vorhandene Druckluftaufbereitungen erfolgen. Die Druckluftversorgung wird an das Handgerät 1 direkt oder über das Steuergerät 40 angeschlossen. Der Bediener stellt bevorzugt am Steuergerät 40 gewünschte Betriebsparameter für Druck und Stichfrequenz ein und kann diese auch abspeichern. Über den Schalter 43 wird das Handstück 1 in Betrieb genommen, und die Anwendung wird vom Bediener durchgeführt. Die Druckluftzuführung kann über einen Fußschalter (nicht dargestellt) erfolgen. Hiermit kann die Risikogewichtung bei einem Fehler am Steuergerät 40 herabgesetzt werden, da der Bediener direkt zwangsweise den Ablauf beendet, wenn der Fußschalter nicht mehr betätigt wird.

Die Vorbereitung des Handgerätes 1 für die Anwendung erfolgt durch den Bediener und kann folgenden Arbeitsschritten vorsehen: Anordnen des Moduls 21 am Handgerät 1; Einsetzen des Substanzreservoirs 4; und das Handgerät 1 wird zur Vorbereitung der Anwendung in Betrieb genommen, um den Wirkstoff zur Kanüle 28 zu transportieren. Der Vorgang ist beendet, wenn der Bediener einen Austritt des Wirkstoffes an der Kanülenspitze 27 feststellt.

Das Gehäuse 2 kann zwei Halbschalen gebildet sein, die, wie wahlweise auch weitere oder alle Komponenten des Handgerätes 1, an einer Tragplatte angeordnet sind. Des Weiteren besitzt das Handstück 1 einen Schlauchanschluss für die Hauptdruckluftversorgung und ein Kabelanschluss, dessen Übertragungskabel die Signalverbindung zum Steuergerät 40 herstellt. Kabel und Schlauch werden über die entsprechenden Anschlüsse mit dem Gerät verbunden.

Die Modulaufnahme 20 ist eine passend zum Modul 21 ausgebildete Vertiefung, die den Modulkörper eindeutig bestimmt und lageorientiert aufnimmt, wobei das Modul 21 von vorn entlang der Längsachse eingesteckt wird.

Im Handstück 1 erfolgt die Kopplung der Ausgänge der Pneumatikventile mit den entsprechenden Austrittsöffnungen in der Modulaufnahme 20. In der Modulaufnahme 20 sind entsprechende Abdichtungselemente für die Kopplung des Moduls 21 vorgesehen, die eine verlustfreie Druckluftverbindung mit dem Modul herstellen.

Ein Modulverriegelungsbügel, welcher zur Positionierung und Arretierung des Moduls 21 am Handgerät 1 dient, kann vorgesehen sein. Vor dem eigentlichen Zuführen des Moduls 21 ist der Modulverriegelungsbügel in einer geöffneten Stellung. Hierzu wird der Modulverriegelungsbügel um seinen Drehpunkt nach oben geschwenkt, bis er in der Endposition einrastet. Nach dem Einschieben des Moduls 21 in die Modulaufnahme 20 wird der Modulverriegelungsbügel in Gegenrichtung aus der Endposition zurückgeschwenkt, bis er die Verriegelungsposition erreicht hat. Dabei schiebt der Modulverriegelungsbügel, wenn nötig, mit seiner Kontaktstelle zum Modul 21, das Modul 21 weiter in die Modulaufnahme 20 hinein und verpresst die Modulrückwand mit den Dichtungselementen in der Modulaufnahme 20. Ein Rastelement am Modulverriegelungsbügel kann dafür sorgen, dass der Bügel in Verbindung mit dem Modul 21 in seiner Position fixiert bleibt.

Als Substanzreservoir 4 kann eine handelsübliche Spritze zum Einsatz kommen, zum Beispiel ausgestattet mit einer Luer-Lok-Kopplung. Hierzu wird in einer möglichen Ausführung eine Einmalkanüle auf die Spritze aufgesetzt und der Wirkstoff aus einem beliebigen Vorratsbehälter aufgezogen. Im Anschluss wird die Einmalkanüle von der Spritze entfernt und die im Kolben eingedrehte Kolbenstange herausgeschraubt. Die so vorbereitete Spritze wird mit einer Drehbewegung auf das aus der Sterilverpackung entnommene Modul 21 aufgeschraubt. Bei der Produktion der Module wird darauf geachtet, dass sich die Spitze des Moduls in der 0-Lage befindet und somit eine Verletzungsgefahr durch die hervorstehende Kanüle ausgeschlossen ist. 0-Lage bedeutet, dass die Kanüle 28 in keiner Position aus dem Modul 21 hervorsteht.

Der Modulverriegelungsbügel des Handgerätes 1 wird nach Außen geklappt und das Modul 21 wird durch die entstehende Öffnung in die Modulaufnahme 20 eingesetzt. Anschließend wird der Bügel wieder zurück geklappt, bis eine Rastnase des Bügels an die Aufnahme 20 des Moduls 21 einrastet. Sollte der Bügel seine Endposition nicht erreichen, ist die Nutzung des Handgerätes 1 ausgeschlossen, da das Druckluftsystem (pneumatischer Antrieb) nicht geschlossen ist und so für die Bewegung des Bauteils 29 mit der Kanüle 28 keine Antriebskraft bereitgestellt ist.

Der Einrichtvorgang des Handstückes 1 kann mittels Betätigen einer Taste am Steuergerät 40 eingeleitet werden. Hierauf werden die Schubstange 9 und die Gewindestange 10 so weit vorgefahren, dass der Reservoirkolben 6 rückseitig berührt wird. Im nächsten Schritt wird der Wirkstoff kontinuierlich dosiert, indem die Gewindestange 10 weiter vorfährt, solange der Bediener die dafür vorgesehene Taste am Steuergerät betätigt. Hierfür muss die Spitze des Moduls 21 soweit verdreht werden, dass der maximale Kanülenherausstand in Beziehung zur Austrittsöffnung 26 eingestellt wird und die Kanülenspitze 27 vorn am Modul 21 sichtbar ist. Bei der kontinuierlichen Dosierung werden im Modul 21 die Fluidverbindung 34 und die Kanüle 28 mit Wirkstoff gefüllt, der anschließend bei der Kanülenspitze 27 austritt. Ist das geschehen, ist der Einrichtvorgang abgeschlossen, und die Taste wird losgelassen.

Zum genauen Dosieren ist der elektromotorische Antrieb 15 vorgesehen. Dieser Antrieb 15 sorgt mit der Spindel für ein kontrolliertes Ausdrücken des Substanzreservoirs 4, indem die Umdrehungen des Motors gesteuert und somit der Verfahrweg von Gewindestange 10 und Schubstange 9 vorgegeben wird. Die Dosiermenge wird vom Bediener vorher am Gerät eingestellt. Der Zeitpunkt der Dosierung (Ausgabezeitpunkt) erfolgt zum einen über die Steuerung der Bewegung der Gewindestange 10 und zum anderen über die mechanische Freigabe des Wirkstoffflusses (Fluidsteuereinrichtung 39), in Abhängigkeit von der Position der Kanüle 28. Die mechanische Freigabe erfolgt innerhalb des Moduls 21, indem die Verengung des Verbindungsschlauchquerschnittes in der Fluidverbindung 34 aufgehoben wird.

Es kann umgeschaltet werden zwischen einer Betriebsart, in welcher mehrere Wiederholungen der vorgewählten Frequenz entsprechend vom Gerät automatisch ausgeführt werden, und einer anderen Betriebsart, in welcher Einzelstiche jeweils vom Benutzer ausgelöst werden, zum Beispiel mittels Betätigen eines Hand- oder eines Fußschalters. Eine gewünschte (automatische) Stichfrequenz kann am Steuergerät 40 vorgewählt. Die Stichfrequenz kann zum Beispiel in einem Bereich von etwa 0,1 Hz bis etwa 30 Hz einstellbar sein.

Die gewünschte Stichtiefe wird mit Hilfe der Spitze eingestellt (vgl. Erläuterungen oben). Die Spitze des Handstückes 1 wird auf die Haut aufgesetzt, und das Handstück 1 wird mit Betätigung des Fußschalters 43 in Betrieb genommen. Das Handstück 1 arbeitet mit der eingestellten Frequenz solange wie der Schalter 43 gedrückt bleibt oder das Substanzreservoir 4 entleert ist, d.h. der Reservoirkolben 6 befindet sich in der vorderen Endposition. Diese Position wird über die Steuerung des Handstückes 1 detektiert, und das Handstück 1 wird hierauf abgeschaltet. Über eine gesonderte Taste am Steuergerät 40 wird die Spindel des Handstückes 1 wieder in die Grundposition zurückgefahren, und das Modul 21 kann nach der Entriegelung entnommen und zusammen mit dem Substanzreservoir 4 entsorgt werden.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Modul (21) für ein Handgerät (1) zum Einbringen einer Substanz durch die Haut, mit:
- einem Modulgehäuse (22), das eingerichtet ist, lösbar an ein Handgerät zum Einbringen einer Substanz durch die Haut zu koppeln,
- einer Stecheinrichtung, die in dem Modulgehäuse (22) angeordnet ist und bei der
- ein Bauteil (29) in dem Modulgehäuse (22) entlang einer Bauteilführung zwischen einer ersten und einer zweiten Endstellung verlagerbar ist und
- an dem Bauteil (29) eine Kanülenanordnung (28) mit einer oder mehreren Kanülen aufgenommen sind, derart, dass eine jeweilige Kanülenspitze (27) in zumindest der ersten Endstellung einer Austrittsöffnung (26) des Modulgehäuses (22) außenseitig vorgelagert ist,
- einem Substanzanschluss (35), der an dem Modulgehäuse (22) gebildet und eingerichtet ist, beim Anschließen an das Handgerät an ein druckbeaufschlagtes Substanzreservoir mit der Substanz zu koppeln,
- einer Fluidverbindung (34), über welche die Kanülenanordnung (28) mit dem Substanzanschluss (35) in Verbindung steht, derart, dass die Substanz aus dem Substanzreservoir über den Substanzanschluss (35) in die Fluidverbindung und die eine oder die mehreren Kanülen einspeißbar ist,
- einem Antriebsanschluss (32), der an dem Modulgehäuse (22) gebildet und über welchen eine das Bauteil (29) im Betrieb treibende Antriebskraft einkoppelbar ist, und
- einer der Fluidverbindung (34) zugeordneten Fluidsteuereinrichtung (39), die in dem Modulgehäuse (22) angeordnet und eingerichtet ist, die Fluidverbindung (34) wenigstens dann zu öffnen, wenn das Bauteil (29) in der ersten Endstellung angeordnet ist, und die Fluidverbindung (34) im Vergleich zum Öffnen teilweise oder ganz zu schließen, wenn das Bauteil (29) in der zweiten Endstellung angeordnet ist.

2. Modul (21) nach Anspruch 1, **dadurch gekennzeichnet, dass** die Fluidverbindung (34) in einem Teilabschnitt oder ihre gesamte Länge zwischen dem Substanzanschluss (35) und der einen oder den mehreren Kanülen aus einem flexiblen Material gebildet ist.

3. Modul (21) nach Anspruch 1 oder 2, **dadurch gekennzeichnet, dass** der Substanzanschluss (35) und / oder der Antriebsanschluss (32) an einer Rückwand (23a) des Modulgehäuses (22) angeordnet sind.

4. Modul (21) nach mindestens einem der vorangehenden Ansprüche, **dadurch gekennzeichnet, dass** die Fluidsteuereinrichtung (39) mehrteilig ausgeführt ist.

5. Modul (21) nach mindestens einem der vorangehenden Ansprüche, **dadurch gekennzeichnet, dass** die Fluidsteuereinrichtung (39) einen Klemmmechanismus aufweist, der eingerichtet ist, die Fluidverbindung (34) zum teilweisen oder vollständigen Schließen abzuklemmen.

6. Modul (21) nach mindestens einem der vorangehenden Ansprüche, **dadurch gekennzeichnet, dass** die Fluidsteuereinrichtung (39) mit dem Bauteil (29) in Wirkverbindung steht.

7. Modul (21) nach mindestens einem der vorangehenden Ansprüche, **dadurch gekennzeichnet, dass** die Fluidverbindung (34) gegenüber einem rückseitigen Kanülenanschluss (33) in Längsrichtung der zugeordneten Kanüle verläuft.

8. Modul (21) nach mindestens einem der vorangehenden Ansprüche, **dadurch gekennzeichnet, dass** an dem die Kanülenanordnung (28) tragenden Bauteil (29) ein Kolben (30) angeordnet ist, welcher sich beim Verlagern zwischen der ersten und der zweiten Endstellung zumindest abschnittsweise in einer Kolbenführung (31) bewegt.

9. Modul (21) nach mindestens einem der vorangehenden Ansprüche, **dadurch gekennzeichnet, dass** der Antriebsanschluss (32) einen pneumatischen Anschluss aufweist.

10. Modul (21) nach mindestens einem der vorangehenden Ansprüche, **dadurch gekennzeichnet, dass** das Modulgehäuse (22) mehrteilig mit einem Modulgehäusekörper (23) und einer Modulgehäusespitze (24) ausgeführt ist, die am Modulgehäusekörper (23) und relativ hierzu verlagerbar angeordnet ist.

11. Modul (21) nach mindestens einem der vorangehenden Ansprüche, ausgeführt als Einwegmodul.

12. Artikel, mit einem in einer Sterilverpackung aufgenommenen Modul (21) nach mindestens einem der vorangehenden Ansprüche.

13. Anordnung, mit:
- einem Handgerät (1) zum Einbringen einer Substanz durch die Haut und
- einem Modul (21) nach mindestens einem der Ansprüche 1 bis 11,
wobei das Modul (21) lösbar an einem Modulanschluss des Handgerätes (1) angeordnet ist, derart, dass der Substanzanschluss (32) des Moduls (21) an ein druckbeaufschlagtes Substanzreservoir des Handgerätes (1) mit der durch die Haut einzubringenden Substanz und der Antriebsanschluss (35) des Moduls (21) an eine Antriebseinrichtung des Handgerätes (1) funktionell koppeln.

14. Anordnung nach Anspruch 13, **gekennzeichnet** eine Steuereinrichtung aufweist, die an eine Druckeinrichtung, welche das Substanzreservoir mit einem Betriebsdruck beaufschlagt, und die Antriebseinrichtung koppelt und welche eingerichtet ist, jeweilige Steuersignale an die Druckeinrichtung und die Antriebseinrichtung zeitlich koordiniert abzugeben.

15. Anordnung nach Anspruch 14, **dadurch gekennzeichnet, dass** zumindest Teilkomponenten der Steuereinrichtung in einem Handgerätegehäuse angeordnet sind.
